# EUROPEAN PATENT APPLICATION

(11) **EP 2 532 242 A1**
(43) Date of publication of application: **12.12.2012**
(21) Application number: 12169450.9
(22) Date of filing: 25.05.2012
(51) Int. Cl.: A01P 1/00, A01N 43/40, A61K 31/44, A61L 2/16

(54) **Use of bispyridiniumalkanes for killing spores**

(30) Priority: 10.06.2011 DE 102011077432
(71) Applicant: L'AIR LIQUIDE, SOCIETE ANONYME POUR L'ETUDE ET L'EXPLOITATION DES PROCEDES GEORGES CLAUDE, 75007 Paris (FR); SCHÜLKE & MAYR GMBH, 22851 Norderstedt (DE)
(72) Inventor: Steinhauer, Katrin, 22459 Hamburg (DE); Pauselius, Irene, 22846 Norderstedt (DE); Krellenberg, Alexandra, 23566 Lübeck (DE)
(74) Representative: Conan, Philippe Claude

(57) **Abstract**

It has been found according to the invention that spores can be killed with bispyridiniumalkanes. The invention relates, according to a first aspect, to the use of bispyridiniumalkanes for killing spores. According to a second aspect, the invention relates to bispyridiniumalkanes for use for killing spores. According to a third aspect, the invention relates to a disinfectant which comprises one or more bispyridiniumalkanes for use for killing spores. In a fourth aspect, the invention relates to a disinfectant which comprises one or more bispyridiniumalkanes for killing spores. According to a fifth aspect, the invention relates to the use of bispyridiniumalkanes in the production of a disinfectant for preventing contamination of the disinfectant with spores. In all embodiments of the invention, octenidine dihydrochloride is preferred as bispyridiniumalkane. If the disinfectant comprises alcohol, then the presence of one or more aromatic alcohols is obligatorily prescribed.

## Description

The present invention relates to the use of bispyridiniumalkanes for killing spores. Furthermore, the invention relates to bispyridiniumalkanes for use for killing spores. The invention also relates to a disinfectant which comprises one or more bispyridiniumalkanes for use for killing spores. In addition, the invention relates to the use of a disinfectant which comprises one or more bispyridiniumalkanes for killing spores. Finally, the invention relates to the use of bispyridiniumalkanes in the production of a disinfectant for preventing contamination of the disinfectant with spores. If the disinfectant comprises alcohol, then the presence of one or more aromatic alcohols in the disinfectant is obligatorily prescribed.

The reliable killing of bacteria plays a decisive role in a multitude of areas. Some bacteria form persistent stages (so-called spores), in which the complete metabolism is brought to a standstill. In this state, the bacteria are able to withstand even extreme environmental conditions and sometimes survive for several years to decades. Suitable conventional aggressive chemical active ingredients for reliably killing spores are, inter alia, peracetic acid, hydrogen peroxide, ozone, sodium hypochlorite and mixtures of fruit acids with hydrogen peroxide.

As a reaction to a hunger state, some bacteria form so-called endospores. Endospores is the term used to refer to a survival form which is formed within an organism or a cell, which differentiates them from exospores. Aerobic and anaerobic, endospore-forming, Gram-positive bacteria of the genera *Bacillus* spp. and *Clostridium* spp. have in past years become the focus of attention, particularly with regard to disinfection in the hospital sector. Bacteria of this type of the genus *Bacillus* include *Bacillus antracis,* the pathogen of anthrax. Members of the genus *Clostridium* are *Clostridium perfringens* (causes gaseous gangrene), *Clostridium botulinum* (causes botulism) and *Clostridium* tetani (causes tetanus).

According to the list of disinfectants and disinfection methods recognized and tested by the Robert Koch Institute (the central facility in the Federal Republic of Germany in the field of disease monitoring and prevention) (status 31 May 2007, 15th edition), it is necessary, for example for killing spores of *Clostridium tetani,* to destroy the spores by combustion or special (chemo)thermal disinfection methods (e.g. at superatmospheric pressure, 134°C, 1 h). However, for certain materials which may be carriers of spores, combustion, the use of the specified conventional chemical active ingredients or (chemo)thermal treatment under superatmospheric pressure are not suitable, for example in the case of disinfection of skin, such as in the case of disinfection of hands, mucosa or wounds.

Hand disinfection is one of the essential methods for breaking infection chains in hospitals and in the form of "Surgical hand disinfection" (European Standard EN 12791), is an essential part of the measures for preventing wound infections in the case of surgical interventions. It is generally known that bacterial spores on the skin cannot be killed by customary alcohol-based disinfectants. Also, according to Hübner, N―O., Belo, H. and Kramer, A., GMS Krankenhaushyg. Interdiszip. 2006; 1(1):Doc 13 (ISSN 1863-5245), sporicidal hand disinfectants are virtually nonexistent or are very aggressive. The authors recommend that the surgeon removes loosely adhering spores from his hands by washing and then disinfects the hands in the normal manner using an alcoholic hand disinfectant. Hand washing leads to a lowering of the spore load. Wallhäuβer's Praxis der Sterilisation, Desinfektion, Antiseptik und Konservierung [Practice of sterilization, disinfection, antisepsis and preservation], Kramer, A. and Assadian, O. (Eds.), Georg Thieme Verlag 2008, Stuttgart, New York, describes how didecyldimethylammonium chloride and benzalkonium chloride prevent the germination of spores. For benzalkonium chloride, however, it is reported that spores are not killed.

It was an object of the invention to provide active ingredients for disinfectants which kill spores. In addition, the aim was to provide disinfectants which comprise these active ingredients and which likewise kill spores.

This object is achieved according to a first aspect through the use of bispyridiniumalkanes for killing spores.

According to a second aspect, the invention relates to bispyridiniumalkanes for use for killing spores.

According to a third aspect, the invention relates to a disinfectant which comprises one or more bispyridiniumalkanes for use for killing spores.

In a fourth aspect, the invention relates to the use of a disinfectant which comprises one or more bispyridiniumalkanes for killing spores.

According to a fifth aspect, the invention relates to the use of bispyridiniumalkanes in the production of a disinfectant for preventing contamination of the disinfectant with spores. In other words, the fifth aspect relates to a process for producing a disinfectant to which bispyridiniumalkane is added to reduce or prevent contamination with spores.

The as yet unpublished application DE 10 2010 054 137.0 from the applicant describes the use of aliphatic alcohols in the production of a disinfectant for preventing contamination of the disinfectant with bacterial spores. In addition, disinfectants with a content of aliphatic alcohol which are effective against bacterial spores are described. These disinfectants can comprise, for example, bispyridiniumalkanes as antimicrobial active ingredients. However, it is not disclosed in DE 10 2010 054 155.9 that bispyridiniumalkanes are able to kill spores.

If the disinfectant according to the present invention comprises alcohol, then, according to the invention, the presence of one or more aromatic alcohols is obligatorily prescribed. This means that, according to the present invention, disinfectants are excluded which only comprise one or more aliphatic alcohols as alcohol, as is described in DE 10 2010 054 155.9. Thus, if - besides bispyridiniumalkanes - aliphatic alcohol is also present in the disinfectant, then the presence of aromatic alcohol is also prescribed.

The aliphatic alcohol (in the event of its presence in the disinfectant according to the invention, the presence of aromatic alcohol is not only preferred, but obligatory) is, as disclosed in DE 10 2010 054 155.9, preferably a C₁― to C₆―alkyl alcohol, such as, for example, methanol, ethanol, propanol, butanol and mixtures thereof, in particular ethanol and propanol and mixtures thereof.

In an alternatively preferred embodiment, however, the presence of aliphatic alcohols (as are obligatory according to DE 10 2010 054 155.9) is excluded altogether in the disinfectant according to the invention.

The invention is based inter alia on the fact that it has surprisingly been found that the ability of spores to survive upon contact with bispyridiniumalkanes is severely limited (RF up to > 3 log), even without having to use elevated temperature. Hitherto, it had been assumed that the aforementioned conventional aggressive chemical active ingredient and/or (chemo)thermal methods are automatically required for the killing of spores.

The sporicidal effect of bispyridiniumalkanes was therefore also surprising because it was known from the aforementioned prior art that although quaternary ammonium compounds such as benzalkonium chloride and didecyldimethylammonium chloride prevent the germination of spores, they do not kill them. By contrast, the examples of the present application demonstrate that bispyridiniumalkanes exhibit true sporicidity.

According to the present invention, killing of spores thus means that spores are controlled and thereby actually killed, and not only the germination of the spores is prevented - as described for benzylkonium chloride and didecyldimethylammonium chloride. Spores killed according to the invention are preferably derived from anaerobic or aerobic bacteria. These are preferably endospores. They are preferably spores derived from bacteria of the genera *Clostridium* and *Bacillus.* The bacteria are particularly preferably *Clostridium sporogenes, Clostridium di fficile, Clostridium tetani, Clostridium botulinum, Clostridium perfringens, Clostridium novyi, Clostridium histolyticum, Clostridium sordellii, Clostridium ramosum, Clostridium innocuum, Clostridium septicum* or *Bacillus subtilis, Bacillus anthracis, Bacillus cereus, Bacillus licheniformis, Bacillus circulans, Bacillus coagulans.*

Surprisingly, the killing according to the invention of spores takes place here even at a temperature of less than 60°C, preferably less than 50°C, in particular less than 40°C, for example 15°C to 35°C, such as 20°C to 30°C.

### 1. Bispyridiniumalkane

According to the invention, one or more bispyridiniumalkanes are used. According to the invention it is possible to use all suitable bispyridiniumalkanes. The bispyridiniumalkane used according to the invention is preferably a bis[4-(substituted-amino)-1-pyridinium]alkane of the general formula I or II 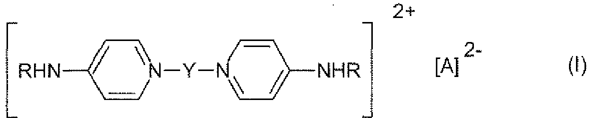 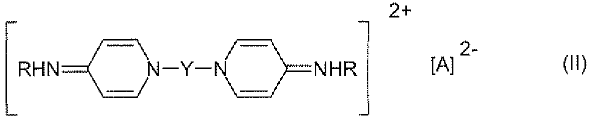 where Y is an alkylene group having 4 to 18 carbon atoms, R is an alkyl group having 6 to 18 carbon atoms or a cycloalkyl group having 5 to 7 carbon atoms or the phenyl radical, which may be substituted by a halogen atom, and A is one or more anions. Octenidine disulfate is suitable. In all embodiments of the invention, N,N'-(1,10-decanediyldi-1[4H]-pyridinyl-4-ylidene)bis(1-octanamine) dihydrochloride (octenidine dihydrochloride, hereinbelow octenidine for short) is particularly preferred as bispyridiniumalkane.

A typical amount of bispyridiniumalkane is at least 0.001% by weight, such as 0.02 to 5% by weight, preferably 0.03 to 3% by weight, in particular 0.04 to 0.5% by weight, such as about 0.1% by weight, or about 0.05% by weight.

Besides the obligatorily prescribed bispyridiniumalkane as sporicidal active ingredient, the disinfectant produced according to the invention comprises, in one preferred embodiment, one or more of the following optional components:
- one or more aromatic alcohols,
- one or more surfactants,
- one or more solvents and/or
- one or more active ingredients and/or auxiliaries.

### 2. Aromatic alcohol

Besides the obligatorily prescribed bispyridiniumalkane effective against spores, preferably one or more aromatic alcohols are present in disinfectants produced according to the invention. The presence of one or more aromatic alcohols, however, is obligatorily prescribed if the disinfectant comprises one or more aliphatic alcohols. If the disinfectant comprises aromatic alcohol as well as bispyridiniumalkane, then the presence of aliphatic alcohol is optional.

Exemplary aromatic alcohols are selected from i) aryloxyalkanols, ii) arylalkanols and iii) oligoalkanol aryl ethers.
(i) Aryloxyalkanols used according to the invention have the formula Ar―O―(CHR)ₙ―OH where R = independently H (when n ≥ 2) or C₁― to C₆―alkyl, where n is an integer and preferably 2 to 10, more preferably 2 to 6 and in particular 2 or 3. While the group Ar can be a ring-substituted or unsubstituted aryl group, unsubstituted aryl, e.g. phenyl or naphthyl, are preferred. Exemplary aryloxyalkanols used according to the invention are phenoxyethanol and phenoxypropanols. Preferred phenoxypropanols are 1―phenoxypropanol―2, 2-phenoxypropanol-1 or mixtures thereof, and also 3-phenoxypropanol-1.
(ii) Arylalkanols used according to the invention have the formula Ar―(CHR)ₙ―OH where R = independently H or C₁― to C₆―alkyl, where n is an integer and preferably 1 to 10, more preferably 1 to 6 and in particular 1, 2, 3 or 4. While the group Ar can be a ring-substituted or unsubstituted aryl group, unsubstituted aryl, e.g. phenyl or naphthyl, are preferred. Exemplary arylalkanols are 3―phenylpropanol―1, phenylethyl alcohol, veratryl alcohol (3,4-dimethoxyphenylmethyl alcohol), benzyl alcohol and 2―methyl―l-phenyl―2― propanol.
(iii) Oligoalkanol aryl ethers include, for example, phenoxy-di-, -tri- and -oligoethanol and phenoxy-di-, -tri- and -oligopropanol.

In this connection, preference is given to an embodiment in which the aromatic alcohol is phenoxyethanol.

A preferred amount of aromatic alcohol (in particular phenoxyethanol) in the disinfectant is 0.1 to 10% by weight, preferably 0.3 to 8% by weight, 0.5 to 5% by weight, in particular 1 to 3% by weight, such as about 2.0% by weight, of aromatic alcohol.

### 3. Surfactant

As optional constituent in the disinfectants produced according to the invention, cationic, anionic, amphoeric and/or nonionic surfactants are present, preferably amphoteric or nonionic surfactant.

As nonionic surfactant, all suitable nonionic surfactants can be used, preference being given to (i) (fatty) alcohol polyalkoxylates, (ii) sorbitan esters, (iii) alkyl glycosides (in particular alkyl polyglucosides), (iv) amine oxides and (v) ethylene oxide/propylene oxide block copolymers.

The (i) alcohol polyalkoxylates include fatty alcohol alkoxylates, e.g. isodecyl ethoxylates with different fractions of ethylene oxide, isotridecyl ethoxylates, polyethylene glycol ethers of stearyl, lauryl and cetyl and oleyl alcohol. Here, the alcohols may have been alkoxylated with ethylene oxide, propylene oxide or any desired mixtures of ethylene oxide and propylene oxide. Alcohol polyalkoxylates are known inter alia under the names Lutensol®, Marlipal®, Marlox®, Brij® and Plurafac®. As nonionic surfactant, particular preference is given to lauryl alcohol ethoxylates.

Furthermore, the nonionic surfactants (ii) used are sorbitan esters, which are mostly present as oleates, stearates, laurates and palmitates and which are referred to as polysorbates (e.g. Tween®).

Moreover, the nonionic surfactant may be a (iii) alkyl glycoside, such as an alkyl glucoside (i.e. an alkyl glycoside of glucose), more preferably a C₈― to C₂₀― alkylpolyglucose, in particular a C₈― to C₁₆― alkylpolyglucose of a fatty alcohol, preference being given to a laurylpolyglucose, a decylpolyglucose or a mixture thereof. The carbon chain length is 8 to 16 atoms in the case of cocoylpolyglucose, 12 to 16 atoms in the case of laurylpolyglucose, and likewise 8 to 16 carbon atoms in the case of decylpolyglucose.

A typical amount of alkyl glycoside is 0.03 to 10% by weight, preferably 0.06 to 5% by weight, in particular 0.1 to 2% by weight.

According to the invention, as (iv) amine oxide it is in principle possible to use all suitable amine oxides. The amine oxides, which are N-oxides of tertiary amines, include aliphatic amine oxides, cyclic amine oxides (such as N-alkylmorpholine oxide) and aromatic amine oxides (such as pyridine N-oxides). In a preferred embodiment, the amine oxide has the general formula

R¹R²R³N-O,

in which R¹ is methyl, ethyl or 2-hydroxyethyl, R² is methyl, ethyl or 2-hydroxyethyl, R¹ and R² together may be morpholine, R³ is alkyl having 8 to 18 carbon atoms or R⁴CONH (CH₂) ₙ, where R⁴ is alkyl having 8 to 18 carbon atoms and n is in the range from 1 to 10, preferably 1 to 5, more preferably 2 to 4, and in particular 3, and may be 2-hydroxyethyl condensed with 1 to 2000 ethylene oxide units, ethylene oxide/propylene oxide units or propylene oxide units.

Exemplary amine oxides are cocamidopropylamine oxide, N-cocomorpholine oxide, decyldimethylamine oxide, dimethylcetylamine oxide, dimethylcocamine oxide, dimethyl-hydr. tallow-amine oxide, dimethyllaurylamine oxide, dimethylmyristylamine oxide, (2-hydroxyethyl)-cocamine oxide and oleamine oxide. See also "International Cosmetic Ingredient Dictionary and Handbook", 10th edition 2004, volume 3, pages 2268-2275 (Surfactants-Cleansing Agents).

In one preferred embodiment, the amine oxide is cocamidopropylamine oxide, i.e. R⁴CO is the acyl radical derived from the fatty acids of coconut oil, n = 3, and R1 and R2 are methyl. This product is sold as Rewominox® B 204 by Evonik, Federal Republic of Germany.

A typical amount of amine oxide is 0.03 to 10% by weight, preferably 0.06 to 5% by weight, in particular 0.1 to 2% by weight.

Amphoteric surfactants are likewise suitable as surfactant, for example betaines. Suitable betaines are described in EP 560 114 A2. Particular preference is given to cocamidopropylbetaine. A typical amount of betaine is 0.03 to 10% by weight, preferably 0.06 to 5% by weight, in particular 0.1 to 2% by weight.

Also suitable as surfactant are cationic surfactants, such as quaternary ammonium salts. In principle, according to the invention, it is possible to use all suitable quaternary ammonium compounds. Preferably, the quaternary ammonium compound is a dialkyldimethylammonium salt.

Quaternary ammonium salts used according to the invention are represented by the formula [R¹R²R³ (CH₃) N]⁺ [X] ⁻, where R¹ to R³ may be identical or different and are selected from C₁― to C₃₀―alkyl, aralkyl, alkenyl and mixed groups, which may have one or more atoms selected from O, S, N and P, where R¹ to R³ are for example C₈― to C₁₈―alkyl, benzyl or methyl, preferably C₉- to C₁₈―alkyl, benzyl or methyl, such as C₁₆-alkyl, benzyl or methyl. X is an anion (of an inorganic or organic acid). In this connection, both anion and cation of the quaternary ammonium salt may be polyvalent ions, which gives rise to a stochiometry [A (ⁿ⁺) ] m [K^{(m+)} ] _{n.}

Suitable quaternary ammonium salts according to the invention are all quaternary ammonium salts of the aforementioned formula known in the prior art, as are disclosed, for example, in WO 00/63337, to which reference is hereby made. However, preference is given to using dialkyldimethylammonium salts, for example dialkyldimethylammonium chlorides, the alkyl chains of which are selected independently of one another from C₈― to C₁₈―alkyl, preferably C₉― to C₁₈―alkyl, such as C₁₆―alkyl. In the dialkyldimethylammonium salts, one of the methyl groups may be an alkoxylated, for example ethoxylated, hydromethyl group.

Quaternary ammonium salts used preferably according to the invention are compounds of the formulae [R¹N (CH₃) ₃] ⁺ [X] ⁻, [R¹R²N (CH₃) ₂] ⁺ [X]⁻ and [R¹R²R³ (CH₃) N] ⁺ [X]⁻ , where R¹ to R³, independently of one another, are selected from C₈― to C₁₈―alkyl and ― (CH₂―CH⁴O) ₙ―R⁵, where n is a number from 1 to 20, preferably 1 to 5, and R⁴ and R⁵, which may be identical or different, are H and/or C₁― to C₄-alkyl, preferably H.

Exemplary anions and classes of anions of the quaternary ammonium salts used are hydroxide, sulfate, hydrogensulfate, methosulfate, ethosulfate, lauryl sulfate, lauryl ether sulfate, cellulose sulfate, sulfamate, halide (fluoride, chloride, bromide, iodide), nitrite, nitrate, carbonate, hydrogencarbonate, phosphate, alkyl phosphate, metaphosphate, polyphosphate, thiocyanate (rhodanide), carboxylic acid salt such as benzoate, lactate, acetate, propionate, citrate, succinate, glutarate, adipate, toluenesulfonate (tosylate) and salicylate. Particularly preferred anions are chloride and propionate.

Particular preference is given to using the quaternary ammonium salts mecetronium etilsulfate (hexadecyl-(ethyl)dimethylammonium ethyl sulfate) and benzalkonium chloride.

### 4. Solvent

Moreover, the disinfectant used optionally comprises 4) Solvent. Preferred solvents are glycols and water, and mixtures thereof. A preferred solvent is water. A preferred glycol is propylene glycol.

### 5. Further active ingredients and/or auxiliaries Exemplary further active ingredients and/or auxiliaries which may optionally be present in disinfectants produced according to the invention are skincare additives, refatting agents, perfumes, fragrances, thickeners, pH regulators, humectants and dyes. These are inter alia:

- polyols, which act as skincare additives, refatting agents and humectants, such as glycerol, erythritol, 1,2,6-hexanetriol, inositol, lactitol, maltitol, mannitol, methylpropanediol, phytantriol, poly-glycerols, sorbitol and xylitol, with glycerol being particularly preferred,
- glycerol esters, preferably glycerol cocoate, isopropyl myristate, isopropyl palmitate, and triglycerides, which act as refatting agents, and/or
- allantoin, urea and aloe vera, which act as skincare additive.

Moreover, one or more glycerol monoalkyl ethers may be present in the disinfectant which is in accordance with the invention and effective against spores, as are disclosed, for example, in DE 103 56 846 A1. Particular preference is given to 1-(2-ethylhexyl) glycerol ether (Sensiva® SC50) .

The mentioned effect, namely that bispyridiniumalkanes lead over time to the death of spores, is present at the typical pH values of disinfectants which are contemplated according to the invention. Preferred pH values of the disinfectants are in the range from 3 to 9, more preferably 4 to 8, such as 4.5 to 7, for example about 5.5. The desired pH can be adjusted, for example, using sodium lactate, citric acid or NaOH.

The disinfectants produced according to the invention are used in the customary manner on animate surfaces, in particular human skin.

Typically, disinfectants according to the invention are in the form of (alcohol-containing) aqueous solution. According to the invention, however, it is also possible to produce semisolid disinfectants, which are referred to below as semisolid preparations, as are described, for example, in DE 10 2005 045 145 Al.

### 6. Semisolid preparations

Ointments (Latin unguenta) are spreadable preparations which are intended for use by application to or rubbing into the skin. They consist of one or more ointment bases (such as vaseline, wool grease, lanolin, etc.), into which the active ingredient is incorporated. The active ingredient should be dissolved or finely dispersed. In order to increase the solubility, ointments often comprise water or oils. In the case of an ointment, however, the fat/oil fraction is higher than the water fraction.

In ointments according to the invention, the viscosity is generally 500 to 15 000 mPa·s, preferably 1000 to 10 000 mPa · s, measured using a rotary viscometer at 95 s⁻¹ and 20°C.

The disinfectants according to the invention are present for example as aqueous-alcoholic gels (hydrogels). Hydrogels are valued due to their transparency and the non-greasy character. Lipophilic gels (oleogels) are likewise used on account of their aesthetic appearance and their consistency-imparting properties. Gels are intended predominantly for external use and should be applied thinly.

A hydrogel is a mostly translucent mass which is produced with the help of gelatin, tragacanth, carbopol, cellulose derivatives or similar swelling substances with the addition of water and glycerol. As a result of the evaporation of the water, they have a cooling effect.

Emulsion is understood as meaning preparations which are composed of immiscible liquids, e.g. oil and water. A distinction is made between W/O (water in oil) or O/W (oil in water) emulsions and ambiphilic emulsions. By adding an emulsifier, the very fine distribution of the liquids in one another is possible and the emulsions are stable as a result, i.e. the oil and the water do not separate again. Depending on the type of use, emulsions are intended for internal or external use. Emulsions for external use are often referred to as lotions. Here, an oil-in-water emulsion is present.

Preferably, the disinfectant is present as hydrogel. Here, preference is given to an embodiment in which the disinfectant is formulated as a disinfectant that can be applied topically (externally).

According to the invention, the disinfectant can comprise further active ingredients which supplement the effectiveness of bispyridiniumalkanes and can possibly be used in a significantly lower concentration than in the known commercial products. As a result, the disadvantages present can sometimes be significantly reduced. These further active ingredients include: clotrimazole and other locally effective antimycotics, cortisones, tretinoin, benzoyl peroxide, aciclovir, local anaesthetics (e.g. benzocaine, lidocaine, polidocanol etc.), antibiotics, bufexamac etc.).

### 7. Refatting system

In a preferred embodiment, the refatting system in the disinfectant consists of a combination of cetearyl octanoate and isopropyl myristate, or consists of cetearyl octanoate and myristyl alcohol. The preferred concentration of cetearyl octanoate is 0.05 to 5% by weight, more preferably 0.1 to 1% by weight, in particular 0.2 to 0.4% by weight, such as about 0.3% by weight. The preferred amount of isopropyl myristate (or myristyl alcohol) is 0.2 to 5% by weight, in particular 0.5 to 3% by weight, such as 0.8 to 1.2% by weight, for example about 1.0% by weight.

### 8. Exemplary formulations

Exemplary formulations are an antiseptic disinfectant, an antiseptic mouthwash solution, an antiseptic washing lotion, an antiseptic wound gel and an antiseptic wound-washing solution. The composition of these preferred disinfectants is given below. Here, it is preferred in each case that octenidine is present as the sole bispyridiniumalkane:

**Antiseptic disinfectant* (formulation V)**

| (% by wt.) | Preferably | More preferably | Particularly |
|---|---|---|---|
| Bispyridiniumalkane | 0.01 - 1.0 | 0.02 - 0.5 | 0.05 - 0.2 |
| Aromatic alcohol | 0.2 - 10 | 0.5 - 5.0 | 1.0 - 3.0 |
| Amphoteric surfactant | 0.03 - 2.0 | 0.05 - 1.0 | 0.1 - 0.5 |
| Glycerol | 0.05 - 2.0 | 0.1 - 1.0 | 0.3 - 0.6 |
| Purified water | ad 100 | ad 100 | ad 100 |

| | | | |
|---|---|---|---|
| * also comprises skincare additive | | | |

**Antiseptic mouthwash solution* (formulation W)**

| (% by wt.) | Preferably | More preferably | Particularly |
|---|---|---|---|
| Bispyridiniumalkane | 0.01 - 1.0 | 0.02 - 0.5 | 0.05 - 0.2 |
| Nonionic surfactant | 0.2 - 10 | 0.5 - 5.0 | 1.0 - 3.5 |
| Glycerol | 0.05 - 2.0 | 0.1 - 1.0 | 0.3 - 0.6 |
| Purified water | ad 100 | ad 100 | ad 100 |

| | | | |
|---|---|---|---|
| * also comprises aroma, antioxidant, sweetener, buffer system and skincare additive | | | |

**Antiseptic washing lotion* (formulation X)**

| (% by wt.) | Preferably | More preferably | Particularly |
|---|---|---|---|
| Bispyridiniumalkane | 0.03 - 3.0 | 0.05 - 1.5 | 0.1 - 0.5 |
| Nonionic surfactants | 5.0 - 60 | 10 - 50 | 20 - 40 |
| Thickener | 0.05 - 10 | 0.2 - 5 | 0.5 - 2.0 |
| Glycerol | 0.05 - 10 | 0.2 - 5 | 0.5 - 2.0 |
| Purified water | ad 100 | ad 100 | ad 100 |

| | | | |
|---|---|---|---|
| * also comprises acidifier and skincare additive | | | |

**Antiseptic wound qel (formulation Y)**

| (% by wt.) | Preferably | More preferably | Particularly |
|---|---|---|---|
| Bispyridiniumalkane | 0.01 - 0.5 | 0.02 - 0.2 | 0.03 - 0.07 |
| Glycol | 2.0 - 30 | 4.5 - 25 | 5.0 - 15 |
| Thickener | 0.5 - 10 | 1.0 - 5.0 | 1.5 - 4.0 |
| Purified water | ad 100 | ad 100 | ad 100 |

**Antiseptic wound-washing solution (formulation Z)**

| (% by wt.) | Preferably | More preferably | Particularly |
|---|---|---|---|
| Bispyridiniumalkane | 0.01 - 0.5 | 0.02 - 0.2 | 0.03 - 0.07 |
| Glycerol monoalkyl ether | 0.02 - 1.0 | 0.04 - 0.5 | 0.05 - 0.2 |
| Glycerol | 0.5 - 10 | 1.0 - 5.0 | 1.5 - 4.0 |
| Purified water | ad 100 | ad 100 | ad 100 |

In a further preferred embodiment, the disinfectant consists of octenidine in water, i.e. no further constituent is present in the disinfectant.

Moreover, preference is given to an isotonic disinfectant which comprises octenidine and glycerol in aqueous solution, as is disclosed in DE 10 2009 049 504.5.

### 9. Use

The use according to the invention can be a use on inanimate surfaces, for example in the case of surface disinfection or instrument disinfection, or a use on animate surfaces, such as the human skin. The use is preferably on the human skin, particularly preferably on hands, mucosa or a wound.

Particularly preferred uses of the bispyridiniumalkanes and disinfectants are:
- skin disinfectants or rinse solutions for antiseptic measures for skin (such as mucosa, wounds) or internal organs, as described in DE 102 05 883 Al,
- the antisepsis of catheter insertion points, as is described in DE 10 2009 049 506 Al,
- mouthwash solutions and mouth antiseptics, as is **described in** DE 10 2008 011 691 Al and DE 2006 051 981 Al, and
- in combination with ultrasound treatment, wound treatment, preoperative skin disinfection, decontamination of intact skin, surface disinfection and instrument disinfection, as described in DE 10 2009 049 505 Al.

Disinfectants produced according to the present invention can be used for wound antisepsis and hygiene and surgical hand disinfection. Alternatively, the disinfectants are used for surface disinfection.

According to one embodiment, the disinfectant produced according to the invention is used for the treatment of wounds. In this connection, preference is given to selecting an emulsifier-free formulation which includes a high degree of moisturizing factors (e.g. a gel). In addition, the sporicidal effect of octenidine is of therapeutic benefit for example for wounds contaminated with spores (e.g. the cutaneous anthrax pathogen *Bacillus antracis).* As a result of the demonstrated sporicidal effect of octenidine, the incidence of infection is positively influenced.

The examples below show that according to the invention it is also possible to control spores which, on account of their storage in aliphatic alcohols, are adapted to a spore-hostile environment.

In the investigations into sporicidity in accordance with EN 13704, the membrane filtration method was used. In the membrane filtration method, the sample treated with the biocide (in this case bispyridiniumalkane) is filtered through a sterile membrane filter (pore size 0.45 µm) and washed. The filter is then placed on an agar plate and incubated. As a result of this method, any active substances still adhering to the spore wall are rinsed away, and a sporicidal effectiveness of the tested biocide can thus be detected. In the case of a biostatic effectiveness, as is described as mentioned for benzalkonium chloride or didecyldimethylammonium chloride, by contrast, a germination is prevented only by the adhesion of the biocide to the spore wall.

### Examples

### 1. Method A: Quantitative suspension experiment in accordance with EN 13704

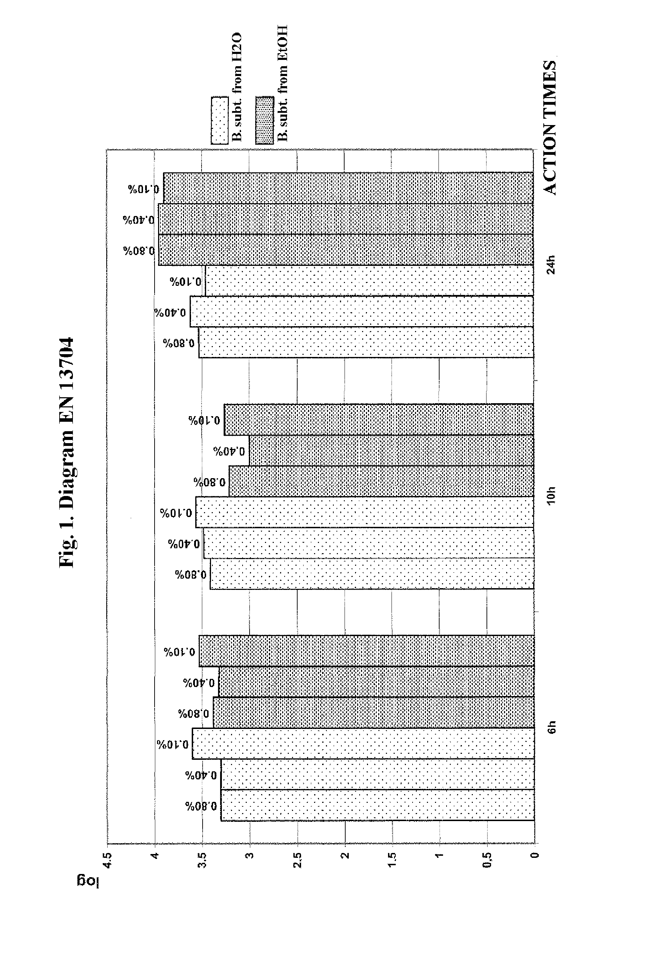

To demonstrate the sporicidal effectiveness of octenidine dihydrochloride (cf. Figure 1), experiments were carried out in accordance with EN 13704 (cf.: European Committee for standardization (2002): European Standard EN 13704: Chemical disinfectants - Quantitative suspension test for evaluation of sporicidal activity of chemical disinfectants used in food, industrial, domestic and institutional areas ― Test method and requirements (phase 2, step 1)). In this, the membrane filtration method was used as the method for deactivating the active substance after the stated action times. With the help of the malachite green coloration, adequate sporulation of the spore suspension used was verified at the start of the experiments by means of light microscopy.

### 2. Method B: Concentration test spores of Bacillus subtilis

### 2.1 Instruments, nutrient media and spores used:

- fixed pipettes 1 ml; 500 µl, and 100 µl
- sterile glass pipettes 10 ml
- sterile spatulas
- sterile Schott bottles
- water bath 30°C
- CSA-agar + TLSH (Tween 80 3%; lecithin 0.3%; saponin 3%, histidine 0.1%)
- *Bacillus subtilis* ATCC 6633 stored in a) H₂O or b) ethanol

Spore samples are provided commercially, sometimes as ethanolic solution (40% ethanol in water).

### 2.2 Diluent:

**Germination medium: + TLSH:**

| | **For 1 litre:** |
|---|---|
| 0.3 M saccharose | 102.7 g |
| 10 mM tris―HCl (pH 7.5) | 10 ml of the 1M stock solution |
| 10 mM MgSO₄ × 7 H₂O | 2.46 g |
| 30 mM CaCl₂ × 6 H₂O | 6.57 g |
| 8 mM L-alanine | 0.71 g |
| (25 µg/ml lysozyme) | 100 µl of the 250 mg/ml stock (if stated) |

### 2.3 Test suspension:

The germ count of the ordered spore solution is ascertained prior to the start of the experiment by means of plating out and counting and, if appropriate, the spore suspension is diluted. On the day of the experiment, a starting germ count (SGC) is again generated from the spore suspension used. By means of spore counting under the microscope in a Helber chamber, a comparison is drawn between spores which are actually present and the number of germinating spores. The starting spore count should be ca. 1 × 10⁷ CFU/ml. With the help of the malachite green coloration, adequate sporulation of the spore suspension used was verified by means of light microscopy.

### 2.4 Action times:

Samples are taken regularly over several weeks. The experimental solutions stand in the dark at room temperature during the action times.

### 2.5 Procedure:

In each case 50 ml per solution to be investigated are pipetted into a sterile Schott bottle, inoculated with 50 µl of spore suspension and incubated at room temperature. Additionally, per test suspension, a Schott bottle containing 50 ml of H₂O is inoculated with 50 µl of test suspension.

As blank value, after brief mixing, in each case 1 ml of the mixture is immediately pipetted to 9 ml of germination medium with lysozyme (if stated) and/or 9 ml of germination medium without lysozyme. These tubes are incubated in a water bath for 15 min at 30°C and then further diluted in germination medium without lysozyme such that 0.5 ml of the 10⁰ and in each case 0.1 ml of the 10⁰, 10⁻¹ dilution can be streaked out. The other samplings are made similarly on the days stated above.

### 2.6 Incubation and evaluation:

The plates are incubated at 36°C for 24 h.

### 2.7 Validation:

To check the nontoxicity of the diluent, 0.1 ml of the 10⁻⁴ dilution of the spore suspension is pipetted to 9.9 ml of germination medium and incubated in a water bath for 15 min at 30°C. 0.5 ml and 0.1 ml are then streaked out.

### 2.8 Lysozyme test:

In order to exclude false negative results, a lysozyme test is carried out in parallel (according to D. L. Popham, J. Helin, C. E. Costello and P. Setlow, Proc. Natl. Acad. Sci. USA, volume 93, pages 15405 to 15410, December 1996).

Figure 1 shows the results of the quantitative suspension experiment in accordance with EN 13704 (method A) at the action times indicated in the figure. The results show the sporicidal effectiveness of octenidine at different concentrations and action times.

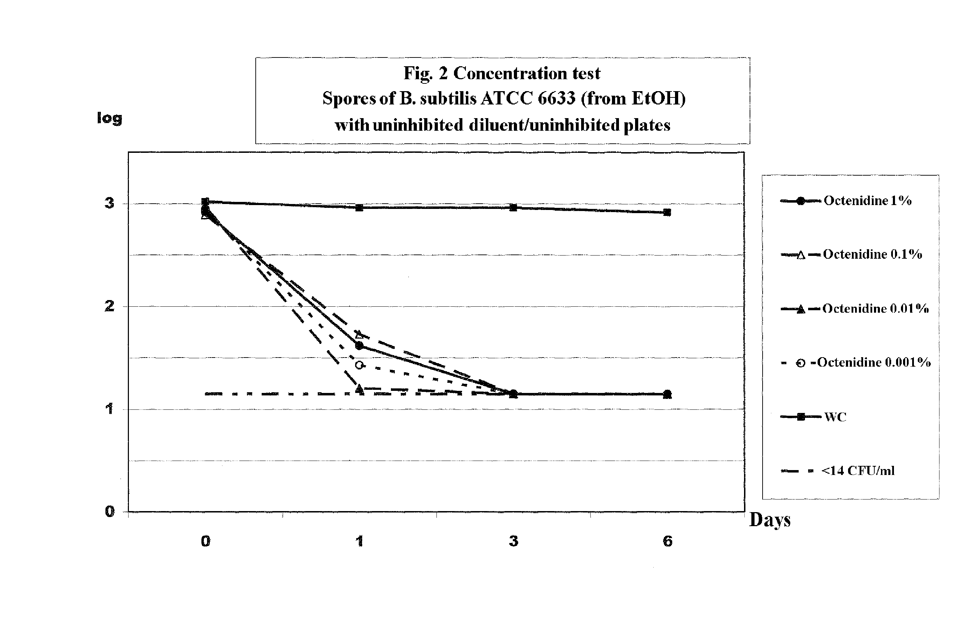

Figure 2 shows results with the concentration test (method B). The results show that octenidine is effective even in a use concentration of 0.001% against *B. subtilis* ATCC 6633, and that this effect can be detected after just one day.

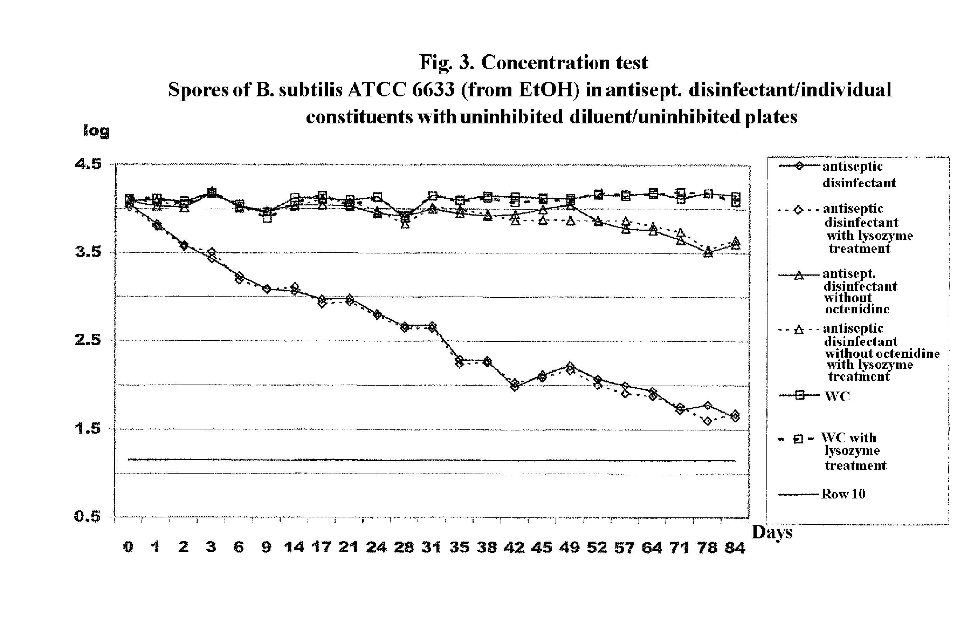

Figure 3 shows the sporicidal effectiveness of an antiseptic disinfectant (with octenidine as bispyridiniumalkane) against *Bacillus subtilis* spores. As comparison, the antiseptic disinfectant was used with identical formulation, but without octenidine, which exhibits no significant sporicidal effectiveness. The water control (WC) served as nonsporicidal reference in this experiment. The spore detection was carried out with and without lysozyme as described under 2. In this experiment, neutralizing agents and also uninhibited agar plates were likewise used as described above for the cultural detection of B. *subtilis* spores. "Row 10" denotes the detection limit.

## Claims

1. Use of bispyridiniumalkanes for killing spores.

2. Use according to Claim 1, **characterized in that** the use takes place at a temperature of less than 60°C, preferably less than 50°C, in particular less than 40°C, for example at a temperature of from 15°C to 35°C, such as 20°C to 30°C.

3. Use according to Claim 1 or 2, **characterized in that** the use concentration of the bispyridiniumalkane is at least 0.001% by weight.

4. Use according to one of the preceding claims, **characterized in that** the bispyridiniumalkane is octenidine dihydrochloride.

5. Use according to one of the preceding claims, **characterized in that** the spores are spores of aerobic or anaerobic bacteria.

6. Use according to one of the preceding claims, **characterized in that** the spores are endospores.

7. Use according to Claim 6, **characterized in that** the endospores are derived from bacteria from the genus *Bacillus* or the genus *Clostridium.*

8. Use according to Claim 7, **characterized in that** the spores are derived from *Clostridium sporogenes, Clostridium difficile, Clostridium tetani, Clostridium botulinum, Clostridium perfringens, Clostridium novyi, Clostridium histolyticum, Clostridium sordellii, Clostridium ramosum, Clostridium innocuum, Clostridium septicum* or *Bacillus subtilis, Bacillus anthracis, Bacillus cereus, Bacillus licheniformis, Bacillus circulans, Bacillus coagulans.*

9. Use according to one of the preceding claims, **characterized in that** the application takes place on the human skin, preferably on hands, mucosa or a wound.

10. Use according to Claim 9, **characterized in that** the use of the bispyridiniumalkane takes place in the form of an antiseptic disinfectant, an antiseptic mouthwash solution, an antiseptic washing lotion, an antiseptic wound gel, a hand disinfectant or an antiseptic wound-washing solution.

11. Use according to Claim 9 or 10, **characterized in that** the wound is a traumatic, acute, chronic, surgical or burn wound.

12. Bispyridiniumalkanes for use for killing spores.

13. Disinfectant which comprises one or more bispyridiniumalkanes for use for killing spores, where, if the disinfectant comprises alcohol, the disinfectant comprises one or more aromatic alcohols.

14. Use of a disinfectant which comprises one or more bispyridiniumalkanes for killing spores,
where, if the disinfectant comprises alcohol, the disinfectant comprises one or more aromatic alcohols.

15. Use of bispyridiniumalkanes in the production of a disinfectant for preventing contamination of the disinfectant with spores.
